(19) Europäisches Patentamt

European Patent Office

Office européen des brevets

(11) **EP 3 889 252 A1**

(12) **EUROPEAN PATENT APPLICATION**
published in accordance with Art. 153(4) EPC

| | |
|---|---|
| (43) Date of publication:<br>**06.10.2021 Bulletin 2021/40** | (51) Int Cl.:<br>***C12N 5/00*** *(2006.01)*     ***C12N 11/08*** *(2020.01)* |
| (21) Application number: **20845090.8** | (86) International application number:<br>**PCT/KR2020/009642** |
| (22) Date of filing: **22.07.2020** | (87) International publication number:<br>**WO 2021/015547 (28.01.2021 Gazette 2021/04)** |

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN** | • **SHIN, Jung Youn**<br>**Daejeon 34122 (KR)**<br>• **KIM, Chanjoong**<br>**Daejeon 34122 (KR)**<br>• **KIM, Minchae**<br>**Daejeon 34122 (KR)**<br>• **KIM, Yunseop**<br>**Daejeon 34122 (KR)**<br>• **KIM, Chang Young**<br>**Daejeon 34122 (KR)**<br>• **NAM, Seung Woo**<br>**Daejeon 34122 (KR)** |
| (30) Priority: **22.07.2019 KR 20190088576**<br>             **21.07.2020 KR 20200090266** | |
| (71) Applicant: **LG Chem, Ltd.**<br>**Seoul 07336, (KR)** | (74) Representative: **Goddar, Heinz J.**<br>**Boehmert & Boehmert**<br>**Anwaltspartnerschaft mbB**<br>**Pettenkoferstrasse 22**<br>**80336 München (DE)** |
| (72) Inventors:<br>• **KIM, Jee Seon**<br>**Daejeon 34122 (KR)** | |

(54) **CELL CULTURE MICROCARRIER, METHOD FOR PRODUCING SAME, AND CELL CULTURE METHOD USING SAME**

(57) The present disclosure relates to a microcarrier that has excellent adhesion to cells and also is easily isolated from cells after culturing, a method for producing the same, and a cell culture method using the same.

【FIG. 1】

Example 1      Example 2      Example 3

EP 3 889 252 A1

**Description**

**[TECHNICAL FIELD]**

CROSS-REFERENCE TO RELATED APPLICATION(S)

This application claims the benefit of Korean Patent Application Nos. 10-2019-0088576 filed on July 22, 2019 and Korean Patent Application No. 10-2020-0090266 filed on July 21, 2020 in the Korean Intellectual Property Office, each of which is incorporated herein by reference in its entirety.

The present disclosure relates to a microcarrier for cell culture, a method for producing the same, and a cell culture method using the same.

**[BACKGROUND OF THE INVENTION]**

Along with the expansion of the fields of biopharmaceuticals and regenerative medicine, the need for the large-scale cell culture technology capable of efficiently producing cells, tissues, microorganisms, and the like is growing.

Adherent cells are cultured using a microcarrier within a 3D bioreactor. A cell, a culture medium, and a microcarrier are put in a bioreactor, and the culture medium is stirred to make contact with the cell and the microcarrier, so that the cells are adhered to the microcarrier surface to culture. The microcarriers used at this time are suitable for the large-scale culture of cells because they provide a high surface area/volume on which cells can adhere and grow.

Currently commercially used microcarriers have a density of about 1.1 to 1.3 $g/cm^3$, and the density of cells is about 1.2 $g/cm^3$. In this case, it is advantageous to adhere cells at early stages of culture within the bioreactor, but at the time of isolating and recovering cells after culturing, a centrifugal separation is difficult, and filtering methods based on the microcarriers and cell size should be utilized. However, in this case, there is a problem that the filter is clogged or the process time is long, and the physical damage and contamination of cells can easily occur, which can lead to loss of cells.

In order to solve the above problems, microcarriers were produced using the properties of a material having a density lower than 1.0 $g/cm^3$ or higher than 1.3 $g/cm^3$, but in this case, there is a disadvantage that the range of densities that can be implemented is limited, and the floatability of microcarriers cannot be adjusted in conformity to the culture conditions.

Therefore, there is a need to develop a new microcarrier that can be more easily isolated at the time of isolating and recovering the microcarriers and the cells after culturing the cells, while adjusting the floatability of particles in conformity to the culture conditions in the aqueous solution.

**[BRIEF SUMMARY OF THE INVENTION]**

The present disclosure provides a microcarrier that has excellent adhesion to cells and also is easily isolated from cells after culturing.

The present disclosure also provides a method for producing a microcarrier that has excellent adhesion to cells and also is easily isolated from cells after culturing.

The present disclosure further provides a cell culture method using the microcarrier that has excellent adhesion to cells and also is easily isolated from cells after culturing.

Provided herein is a microcarrier for cell culture comprising: a foam core containing polystyrene; a primer polymer layer formed on the surface of the foam core; and a cell adhesion-inducing layer formed on the surface of the primer polymer layer, wherein the microcarrier has a density of 0.80 $g/cm^3$ to 0.99 $g/cm^3$.

Also provided herein is a method for producing a microcarrier for cell culture, the method comprising the steps of: heating an aqueous solution containing polystyrene containing a foaming agent; foaming the foaming agent to produce a foam core; and recovering and drying the produced foam core and then applying a primer polymer layer to the surface of the foam core.

Further provided herein is a cell culture method using a microcarrier for cell culture comprising: a foam core containing polystyrene; a primer polymer layer formed on the surface of the foam core; and a cell adhesion-inducing layer formed on the surface of the primer polymer layer, wherein the microcarrier has a density of 0.80 $g/cm^3$ to 0.99 $g/cm^3$.

**[DETAILED DESCRIPTION OF THE INVENTION]**

Hereinafter, a microcarrier for cell culture according to a specific embodiment of the present disclosure, a method for producing the same, and a cell culture method using the same will be described in more detail.

The terms used herein are used only to describe exemplary embodiments, and are not intended to limit the present disclosure. Singular expression includes a plural expression unless they have definitely opposite meanings in the context. It should be understood that the terms "comprise", "include", and "have" as used herein are intended to designate the presence of stated features, numbers, steps, constitutional elements, or combinations thereof, but it should be understood that they do not preclude a possibility of existence or addition of one or more other features, numbers, steps, constitutional elements, or combinations thereof.

Since the present disclosure may be modified in various forms, and may have various embodiments, the following exemplary embodiments are illustrated and described in detail. However, this is not intended to limit the present disclosure to specific embodiments, and the present disclosure should be construed to encompass various changes, equivalents, and substitutions without departing from the technical scope and spirit.

According to one exemplary embodiment of the present disclosure, there can be provided a microcarrier for cell culture comprising: a foam core containing polystyrene; a primer polymer layer formed on the surface of the foam core; and a cell adhesion-inducing layer formed on the surface of the primer polymer layer, wherein the microcarrier has a density of 0.80 $g/cm^3$ to 0.99 $g/cm^3$.

The present inventors have conducted research on microcarriers for cell culture, and confirmed through experiments that when cells are cultured by using a microcarrier comprising: a foam core containing polystyrene; a primer polymer layer formed on the surface of the foam core; and a cell adhesion-inducing layer formed on the surface of the primer polymer layer, wherein the microcarrier has a density of 0.80 $g/cm^3$ to 0.99 $g/cm^3$, the microcarrier and the cells can be easily isolated by using the difference in sedimentation velocity due to gravity after cell culture, while adjusting the fluidity in conformity to the stirring process in an incubator to improve the adhesion to cells, thereby completing the present disclosure.

The microcarrier of the exemplary embodiment is a microcarrier which includes a foam core containing polystyrene, and a primer polymer layer primarily formed on the surface of the foam core, and also includes a cell adhesion-inducing layer secondarily formed on the surface of the primer polymer layer.

In this case, the microcarrier may have a density of 0.80 to 0.99 $g/cm^3$, or 0.85 to 0.99 $g/cm^3$, or 0.90 to 0.99 $g/cm^3$, or 0.95 to 0.99 $g/cm^3$.

When the microcarrier has a density in the above range, the cell and the microcarrier can be easily isolated through the difference in sedimentation velocity due to gravity when isolating and recovering the microcarrier and the cell after culturing the cell.

When the density of the microcarrier exceeds 0.99 $g/cm^3$, the density difference between cells and microcarriers is small, and so centrifugal separation may be difficult during cell isolation and recovery after culture. When the density is less than 0.80 $g/cm^3$, it may be difficult to adhere cells to microcarriers at early stages of culture.

Meanwhile, the foam core of the microcarrier may have a closed cell structure. Usually, the porous structure contains a large number of pores, and the large number of pores may be classified into, for example, two types of closed pores or opened pores. The porous structure may be formed in a structure containing either each of them or both of them.

The closed pores may be pores in which all of the wall surfaces of the pores are formed in a closed structure and thus are not connected to other pores, and are also referred to as closed cells. The opened pores are pores in which at least a part of the wall surfaces of the pores are formed in an opened structure and are connected to other pores, and may be referred to as opened cells.

When the foam core of the microcarrier has a closed cell structure, it is possible to prevent the culture medium from penetrating into the microcarrier during cell culture, and thus, it can be adjusted so that the microcarrier has a density in the above range, and when isolating and recovering microcarriers and cells after culturing the cells, the cells and the microcarriers can be easily isolated through the difference in sedimentation velocity due to gravity.

At this time, when the foam core of the microcarrier has an opened cell structure rather than a closed cell structure, there is a problem that a culture medium penetrates into the microcarrier during culture, thereby increasing the density of the microcarrier.

On the other hand, the culture medium permeation rate can be confirmed by the change in the density of the microcarriers before and after culturing. In other words, it can be confirmed from the following general formula 1, wherein $d_2$ is the density of microcarrier before culturing, $d_3$ is the density of microcarrier after culturing, and $d_m$ is the density of the culture medium. And p means the volume ratio of pores in the microcarrier.

[General Formula 1]

$$\text{Culture medium permeation rate (\%)} = \frac{d_3 - d_2}{d_m} \times \frac{1}{p} \times 100$$

For example, the foam core may have a culture medium permeation rate in the range of 0 vol% to 30 vol% of the internal pore volume at the time of culturing cells at 37°C for 72 hours.

As described above, the foam core has a closed cell structure and can prevent the culture medium from penetrating into the microcarrier during the cell culture. Thus, the permeation rate of the culture medium during cell culture can be adjusted within the above range.

At this time, when the culture medium penetration rate exceeds 30 vol% of the internal pore volume at the time of culturing cells at 37°C for 72 hours, the density of the microcarriers increases, and so centrifugal separation may be difficult at the time of isolating and recovering cells after culturing.

Meanwhile, the foam core may be produced by heating an aqueous solution containing polystyrene containing a foaming agent and then foaming the foaming agent, as in the method of producing a microcarrier described later.

At this time, after increasing the temperature of the aqueous solution containing polystyrene containing the foaming agent, the immersion time in the aqueous solution is adjusted to induce foaming of the foaming agent, thereby adjusting the internal porosity of the foam core.

Meanwhile, the foam core may have pores of 2% to 30%, or 5% to 30%, or 5% to 25% of the volume of the foam therein. That is, the foam core may have an internal porosity of 2% to 30%, or 5% to 30%, or 5% to 25%. The internal porosity means a ratio (or percentage) of the volume of pores formed inside the foam core to the total volume of the foam core. The internal porosity can be measured using various porosimeters or the like by applying a known method without limitation.

Through the adjustment of the internal porosity of the foam core, the floatability of the microcarrier can be adjusted for each cell to be suitable for the culture and isolation process. The floatability of the foam core in the aqueous solution can be adjusted in conformity to the cell culture conditions to increase the adhesion between microcarriers and cells, and it can be easily purified by utilizing the density difference after cell detachment.

On the other hand, the primer polymer layer serves as an adhesive layer capable of introducing a functional polymer onto the surface of polystyrene having no functional group, whereby a polymer layer for cell adhesion is effectively introduced onto the surface of the microcarrier so as to be stably maintained even during culture.

Examples of the primer polymer layer may include, but are not particularly limited to, any one or more one selected from the group consisting of L-dihydroxy phenylalanine (L-DOPA), dopamine, norepinephrine, epinephrine, epigallocatechin, and derivatives thereof as catechol derivatives capable of inducing water-phase adhesion.

Meanwhile, the cell adhesion-inducing layer is composed of cell adhesion materials, which serve to provide a place where transmembrane proteins of cells can bind, and allow adherent cells to stably adhere, spread and culture.

Examples of the polymer forming the cell adhesion-inducing layer may include, but are not particularly limited to, any one or more selected from the group consisting of gelatin, collagen, fibronectin, chitosan, polydopamine, poly L-lysine, vitronectin, peptide containing RGD, lignin, cationic dextran, and derivatives thereof.

The microcarrier includes a primer polymer layer formed on the surface of the foam core containing polystyrene, and by modifying the surface of the microcarrier to be hydrophilic, it can exhibit the effects of being water-dispersed, stably introducing a cell adhesion-inducing layer onto the surface of the primer polymer layer to adjust the floatability of microcarriers in the culture medium, and stably adhering and culturing cells.

Meanwhile, the ratio of the radius of the foam core to the thickness of the primer polymer layer may be 1:0.00001 to 1:0.01, or 1:0.0001 to 1:0.001.

When the ratio of the radius of the foam core to the thickness of the surface coating layer is less than 1:0.00001, the primer polymer layer is too thin relative to the foam core, so the effect of modifying the microcarrier surface to be hydrophilic is insignificant. When the ratio exceeds 1:0.01, the primer polymer layer becomes thicker relative to the foam core, which may reduce the adhesion between cells and microcarriers during the cell culture.

Meanwhile, the microcarrier may have an average diameter of 50 to 800 um, or 100 to 700 um, or 120 to 600 um, or 150 to 500 um. When the average diameter of the microcarrier satisfies the above range, the cell adhesion and culture performance are excellent.

Meanwhile, when the average diameter of the microcarrier is less than 50 um, there is a possibility that the content of the foaming agent is lowered, the foaming efficiency decreases, and the surface area for enabling cell culture is small and thus, the culture efficiency is lowered. When the average diameter exceeds 800 um, there may be a problem that the interaction between adherent cells is reduced, the density of cells in the incubator is reduced, and the cell culture efficiency is reduced, which is not preferable.

Meanwhile, the microcarrier may have a specific surface area of 50 to 2000 $cm^2$/g, or 70 to 1500 $cm^2$/g, or 100 to 800 $cm^2$/g, or 150 to 500 $cm^2$/g. The specific surface area can be measured by applying a known BET (Brunauer-Emmett-Teller) measurement method without limitation.

As described above, the foam core may increase the specific surface area of the microcarrier through volume expansion. When the microcarrier satisfies the specific surface area in the above range, the adhesion between cells and microcarriers is maximized, thus making it more suitable for the large-scale culture of cells.

When the specific surface area of the microcarrier exceeds 2000 $cm^2$/g, the size of the microcarrier becomes small and thus, it may be difficult to isolate microcarriers and cells after culturing the cells. When the specific surface area is less than 50 $cm^2$/g, the number of microcarriers inside the bioreactor is greatly reduced, so that initial microcarrier and cell adhesion and culture efficiency may be reduced.

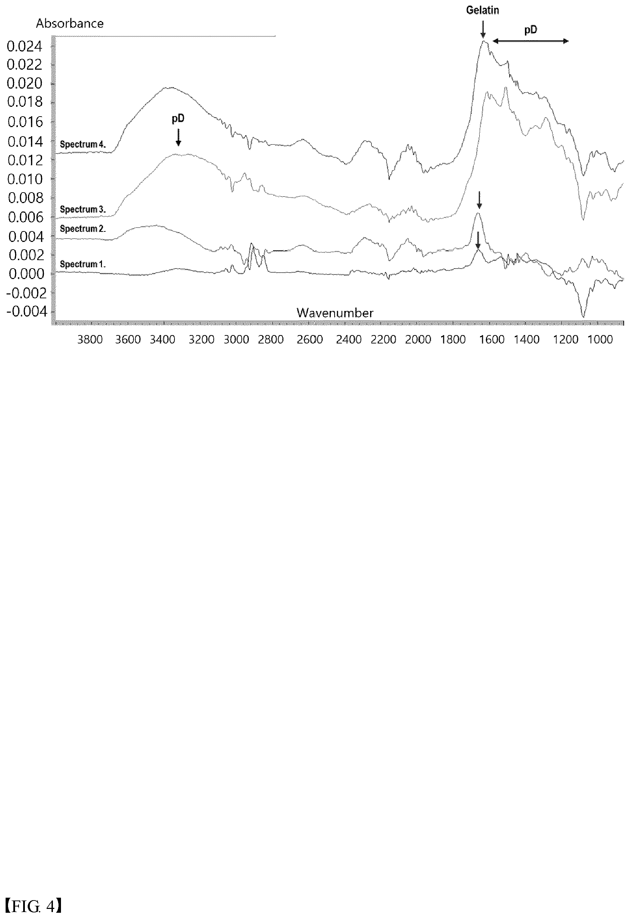

On the other hand, FIG. 3 shows the analysis results of an FT-IR spectrum of a foam containing polystyrene, and specifically, each spectrum of FIG. 3 has the following meaning.

- Spectrum 1: Gelatin on the surface of polystyrene foam
- Spectrum 2: Gelatin on the surface of polystyrene foam to which the polydopamine layer was introduced
- Spectrum 3: Polydopamine on the surface of polystyrene foam
- Spectrum 4: Gelatin and polydopamine layer on the surface of polystyrene foam to which the polydopamine layer was introduced

According to FIG. 3, in the light of the characteristic peak intensity of gelatin based on the amide bond of 1650 $cm^{-1}$ and an amine functional group of the region of 3300-3400 $cm^{-1}$ shown in the spectrum, it can be seen that the cell adhesion-inducing layer (gelatin layer) was more stably introduced into the polystyrene foam to which the primer polymer layer (polydopamine layer) was introduced.

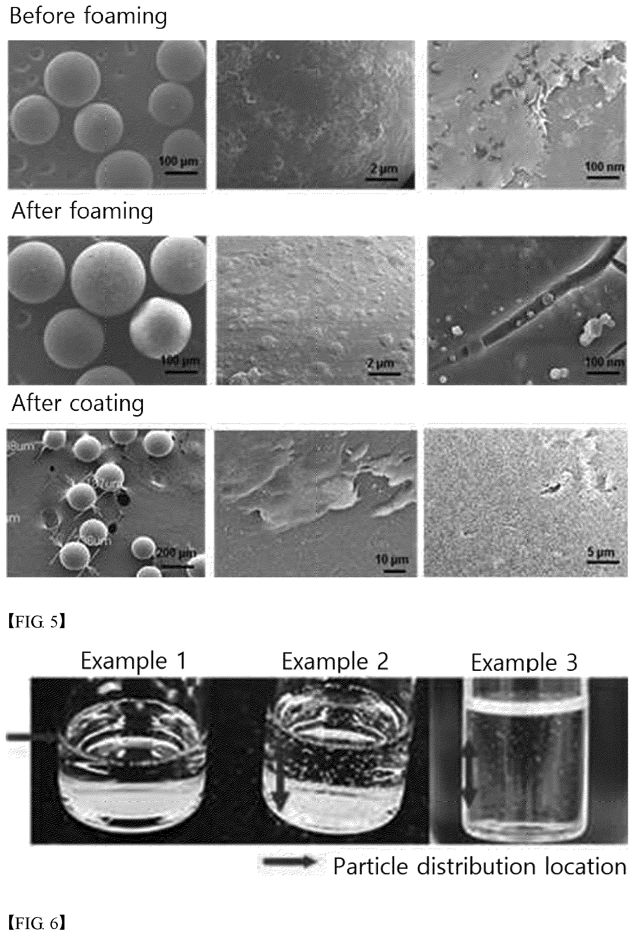

On the other hand, FIG. 4 shows the results of SEM analysis of the polystyrene surface before and after foaming of polystyrene containing a foaming agent and after coating the primer polymer layer. Through the comparison of the shapes of polystyrene corresponding to each case of FIG. 4, it can be confirmed that during the foaming process, the surface roughness of the particles increases and microcracks may occur, but through the primer polymer layer coating process, the cracks on the particle surface were recovered and the roughness was alleviated.

On the other hand, according to another embodiment of the disclosure, there can be provided a method for producing a microcarrier for cell culture, the method comprising the steps of: heating an aqueous solution containing polystyrene containing a foaming agent; foaming the foaming agent to produce a foam core; and recovering and drying the produced foam core and then applying a primer polymer layer to the surface of the foam core.

The polystyrene particles may be obtained by performing suspension polymerization of a polymer of a styrene-based monomer such as styrene, α-methylstyrene, or a styrene-based monomer and a monomer copolymerizable therewith, and examples thereof are not particularly limited.

For example, water, a monomer containing styrene, an initiator, and a surfactant are charged into a reactor to perform primary polymerization, and then the synthesized spherical polystyrene particles are sealed in a reactor, and a foaming agent is injected therein, thereby being able to obtain polystyrene containing a foaming agent.

At this time, examples of the foaming agent may include, but are not particularly limited to, any one or more selected from the group consisting of a physical foaming agent, a chemical foaming agent, an inorganic foaming agent, and mixtures thereof.

For example, the physical foaming agent may be a low-boiling point liquid and an organic solvent, such as butane, pentane, hexane, 2-methylbutane, cyclohexane, neopentane, heptane, isoheptane, benzene, toluene, methyl chloride, trichlorethylene, dichloroethane, methylene chloride, trichlorofluoromethane, Freon 11, Freon 12, Freon 13, Freon 113, Freon 114, and the like.

The method for producing the microcarrier according to the embodiment of the disclosure may include a step of heating the aqueous solution containing polystyrene containing a foaming agent, and foaming the foaming agent to produce a foam core.

At this time, after increasing the temperature of the aqueous solution containing polystyrene containing the foaming agent, the immersion time in the aqueous solution is adjusted to induce foaming of the foaming agent, thereby adjusting the internal porosity of the foam core.

Specifically, the step of heating the aqueous solution containing polystyrene containing a foaming agent may include a step of heat-treating at 50°C to 150°C, or 80°C to 120°C, or 90°C to 110°C for 1 to 10 minutes, or 2 to 8 minutes, or 3 to 5 minutes.

As described above, the foam core may have pores of 2% to 30%, or 5% to 30%, or 5% to 25% of the volume of the foam therein. That is, the foam core may have an internal porosity of 2% to 30%, or 5% to 30%, or 5% to 25%. The internal porosity means a ratio (or percentage) of the volume of pores formed inside the foam core to the total volume of the foam core, and the internal porosity can be measured using various porosimeters or the like by applying a known method without limitation.

Through the adjustment of the internal porosity of the foam core, the floatability of the microcarrier can be adjusted for each cell to be suitable for the culture and isolation process. The floatability of the foam core in the aqueous solution can be adjusted in conformity to the cell culture conditions to increase the adhesion between the microcarriers and the cells, and it can be easily purified by utilizing the density difference after cell detachment.

Meanwhile, the foam core of the microcarrier may have a closed cell structure. When the foam core of the microcarrier has a closed cell structure, it is possible to prevent the culture medium from penetrating into the microcarrier during cell culture, and thus, it can be adjusted so that the microcarrier has a density in the range of 0.80 to 0.99 $g/cm^3$, and when isolating and recovering microcarriers and cells after culturing the cells, the cells and microcarriers can be easily isolated through the difference in sedimentation velocity due to gravity.

On the other hand, the method for producing the microcarrier according to the embodiment of the disclosure may include a step of recovering and drying the produced foam core and then applying a primer polymer layer to the surface of the foam core after the step of foaming a foaming agent to produce a foam core.

Specifically, the step of recovering and drying the produced foam core and then applying a primer polymer layer to the surface of the foam core may include a step of immersing the foam core in the primer polymer solution for 1 to 10 hours, or 2 to 6 hours, or 3 to 5 hours.

Examples of the primer polymer layer may include, but are not particularly limited to, any one or more selected from the group consisting of L-dihydroxy phenylalanine (L-DOPA), dopamine, norepinephrine, epinephrine, epigallocatechin, and derivatives thereof as catechol derivatives capable of inducing water-phase adhesion.

By recovering and drying the produced foam core and then applying a primer polymer layer to the surface of the foam core, and thus, modifying the surface of the microcarrier to be hydrophilic, it can exhibit the effect of being water-dispersed, stably introducing a cell adhesion-inducing layer to adjust the floatability of the microcarrier, and stably adhering and culturing cells.

At this time, the ratio of the radius of the foam core to the thickness of the primer polymer layer may be 1:0.00001 to 1:0.01, or 1:0.0001 to 1:0.001.

Details of the radius of the foam core and the thickness of the primer polymer layer include the contents described above for the embodiment of the disclosure.

On the other hand, the method for producing the microcarrier according to the embodiment of the disclosure may further include a step of coating with a solution containing any one or more selected from the group consisting of gelatin, collagen, fibronectin, chitosan, polydopamine, poly L-lysine, vitronectin, peptide containing RGD, lignin, cationic dextran, and derivatives thereof after the step of recovering and drying the produced foam core and then applying a primer polymer layer to the surface of the foam.

The solution containing any one or more selected from the group consisting of gelatin, collagen, fibronectin, chitosan, polydopamine, poly L-lysine, vitronectin, peptide containing RGD, lignin, cationic dextran, and derivatives thereof can act as an adhesion factor that adheres cells to microcarriers. Thus, when the primer polymer layer is coated with a solution containing the gelatin or the like, the adhesion between cells and microcarriers can be increased, and thus, it can be more suitable for the large-scale culture of cells.

Specifically, the step of coating with a solution containing any one or more selected from the group consisting of gelatin, collagen, fibronectin, chitosan, polydopamine, poly L-lysine, vitronectin, peptide containing RGD, lignin, cationic dextran, and derivatives thereof after the step of recovering and drying the produced foam core and then applying a primer polymer layer to the surface of the foam may include a step in which a material obtained from the step of recovering and drying the produced foam core and then applying a primer polymer layer to the surface of the foam core is immersed in a solution containing any one or more selected from the group consisting of gelatin, collagen, fibronectin, chitosan, polydopamine, poly L-lysine, vitronectin, peptide containing RGD, lignin, cationic dextran, and derivatives thereof for 10 to 20 hours, or 15 to 20 hours, or 17 to 19 hours.

On the other hand, according to another embodiment of the disclosure, there can be provided a cell culture method using a microcarrier for cell culture comprising: a foam core containing polystyrene; a primer polymer layer formed on the surface of the foam core; and a cell adhesion-inducing layer formed on the surface of the primer polymer layer, wherein the microcarrier has a density of 0.80 g/cm$^3$ to 0.99 g/cm$^3$.

Details of the microcarrier for cell culture comprising: a foam core containing polystyrene; a primer polymer layer formed on the surface of the foam core; and a cell adhesion-inducing layer formed on the surface of the primer polymer layer, wherein the microcarrier has a density of 0.80 g/cm$^3$ to 0.99 g/cm$^3$, include the contents described above for the embodiment of the disclosure.

The cells for enabling culture using the microcarrier for cell culture are adherent animal cells, and examples thereof include, but are not particularly limited to, fibroblasts, chondrocytes, mesenchymal stem cells, CHO, HEK 293, vero cells, BHK21, MDCK, and the like.

**[ADVANTAGEOUS EFFECTS]**

According to the present disclosure, a microcarrier that has excellent adhesion to cells and also is easily isolated from cells after culturing can be provided.

In addition, according to the present disclosure, a method for producing a microcarrier that has excellent adhesion to cells and also is easily isolated from cells after culturing can be provided.

Further, according to the present disclosure, a cell culture method using the microcarrier that has excellent adhesion to cells and also is easily isolated from cells after culturing can be provided.

**[BRIEF DESCRIPTION OF THE DRAWING]**

FIG. 1 is the optical microscope observation images of the microcarriers produced in Examples 1 to 3 of the present disclosure.

FIG. 2 shows an SEM analysis result of a cross section after foaming of a microcarrier produced in Example 3 of the present disclosure.

FIG. 3 shows the FT-IR spectrum results of a foam containing polystyrene.

FIG. 4 shows the SEM analysis results of the polystyrene surface before and after foaming of polystyrene containing a foaming agent and after coating a primer polymer layer.

FIG. 5 shows the results of observing the distribution of the microcarriers produced in Examples 1 to 3 of the present disclosure in the culture medium.

FIG. 6 shows the results of cell culture of $1 \times 10^4$ chondrocytes for 3 days using 0.2 g of microcarriers produced in Example 3 and Comparative Example 2 of the present disclosure.

FIG. 7 shows the results of analyzing the cell adhesion shape of the microcarriers produced in Example 3 and Comparative Example 3 of the present disclosure.

Below, the present disclosure will be described in more detail by way of examples. However, these examples are provided for illustrative purposes only, and should not be construed as limiting the scope of the present disclosure to these examples.

**Example 1: Production of microcarriers for cell culture**

An aqueous solution containing foamable polystyrene particles (provided by LG Chem) with an average diameter of 250 um or less was heated at 100°C for 5 minutes, and then water at room temperature was added, and the foaming process was stopped to produce a foam core, which was then recovered, and dried at room temperature.

The dried foam core was immersed in a 1 mg/mL dopamine aqueous solution, and stirred for 4 hours to evenly introduce a polydopamine layer onto the surface of the foam, wherein the thickness of the primer polymer layer was 1 $\mu$m or less. The coated foam was recovered, washed with ethanol at least 3 times, and dried at room temperature for 18 hours. In order to purify particles with a density of 0.8g/cm$^3$ (0.78 - 0.83 g/cm$^3$), separation based on density differences was performed using 75% aqueous ethanol solution and 100% ethanol, and then, placed in a sterile gelatin solution having a concentration of 0.2 w/v, coated for 18 hours, recovered, washed with ethanol, then dried and recovered. By the above method, a microcarrier for cell culture having an average particle size of 500 um and a specific surface area of 150 cm$^2$/g was produced.

**Example 2: Production of microcarrier for cell culture**

An aqueous solution containing foamable polystyrene particles (supplied by LG Chem) with an average diameter of 250 um or less was heated at 100°C for 4 minutes, and then water at room temperature was added, and the foaming process was stopped to produce a foam core, which was then recovered, and dried at room temperature.

The dried foam core was immersed in a 1 mg/mL dopamine aqueous solution, and stirred for 4 hours to evenly introduce a polydopamine layer onto the surface of the foam, wherein the thickness of the primer polymer layer was 1 $\mu$m or less. The coated foam was recovered, washed with ethanol at least 3 times, and dried at room temperature for 18 hours.

Subsequently, it was placed in a sterile gelatin solution having a concentration of 0.2 w/v, coated for 18 hours, recovered, washed with ethanol, then dried and recovered. In order to purify particles with a density of 0.9g/cm$^3$ (0.85 - 0.93 g/cm$^3$), separation based on density differences was performed using 30% aqueous ethanol solution and 70% aqueous ethanol solution. By the above method, a microcarrier for cell culture having an average particle size of 350 um and a specific surface area of 190 cm$^2$/g was produced.

**Example 3: Production of microcarrier for cell culture**

An aqueous solution containing foamable polystyrene particles (supplied by LG Chem) with an average diameter of 250 um or less was heated at 100°C for 3 minutes, and then water at room temperature was added, and the foaming process was stopped to produce a foam core, which was then recovered, and dried at room temperature.

The dried foam core was immersed in a 1 mg/mL dopamine aqueous solution, and stirred for 4 hours to evenly introduce a polydopamine layer onto the surface of the foam, wherein the thickness of the primer polymer layer was 1 µm or less. The coated foam was recovered, washed with ethanol at least 3 times, and dried at room temperature for 18 hours.

Subsequently, it was placed in a sterile gelatin solution having a concentration of 0.2 w/v, coated for 18 hours, recovered, washed with ethanol, dried and recovered. In order to purify particles with a density of 0.95g/cm$^3$ (0.93 - 0.98 g/cm$^3$), separation based on density differences was performed using 20% aqueous ethanol solution and 5% aqueous ethanol solution. By the above method, a microcarrier for cell culture having an average particle size of 300 um and a specific surface area of 210 cm$^2$/g was produced.

The density, internal porosity, average diameter, and specific surface area of the produced microcarriers are summarized in Table 1 below.

[Table 1]

| Category | Example 1 | Example 2 | Example 3 |
|---|---|---|---|
| Density (g/cm$^3$) | 0.8 | 0.9 | 0.95 |
| Internal porosity (%) | 23 | 13 | 9 |
| Average diameter (um) | 500 | 350 | 300 |
| Specific surface area (cm$^2$/g) | 150 | 190 | 210 |

**Comparative Example 1: Production of microcarrier for cell culture**

Non-foaming polystyrene particles having a diameter of 250 µm or less were immersed in water at room temperature for 3 minutes, which was then recovered and dried at room temperature. The dried non-foamed core was immersed in 1 mg/mL dopamine aqueous solution and stirred for 4 hours to evenly introduce a polydopamine layer onto the surface of the non-foamed one, wherein the thickness of the primer polymer layer was 1 µm or less. The coated foam was recovered, washed with ethanol at least 3 times, and dried at room temperature for 18 hours.

Subsequently, it was placed in a sterile gelatin solution having a concentration of 0.2 w/v, coated for 18 hours, recovered, washed with ethanol, then dried and recovered. By the above method, a microcarrier for cell culture having an average particle size of 250 um and a specific surface area of 230 cm$^2$/g was produced.

**Comparative Example 2: Production of microcarrier for cell culture**

Low-density polyethylene particles with a diameter of 212-250 um and a density of 0.96 g/cm$^3$ were immersed in water at room temperature for 3 minutes, which was then recovered and dried at room temperature. The dried low-density polyethylene core was immersed in 1 mg/mL dopamine aqueous solution, and stirred for 4 hours to introduce a polydopamine layer, wherein the thickness of the primer polymer layer was 1 µm or less. The coated particles were recovered, washed with ethanol at least 3 times, and dried at room temperature for 18 hours.

By the above method, a microcarrier for cell culture having an average particle size of 300 um and a specific surface area of 200 cm$^2$/g was produced.

**Comparative Example 3: Production of microcarrier for cell culture**

An aqueous solution containing foamable polystyrene particles (supplied by LG Chem) with a diameter of 250 um or less was heated at 100°C for 3 minutes, and then water at room temperature was added, and the foaming process was stopped to produce a foam core, which was then recovered, and dried at room temperature.

The dried foam core was placed in a sterile gelatin solution at a concentration of 0.2 w/v without introducing a primer polymer layer, coated for 18 hours, recovered, washed with ethanol, then dried and recovered. In order to purify particles with a density of 0.95g/cm$^3$ (0.93 - 0.98 g/cm$^3$), separation based on density differences was performed using 20% aqueous ethanol solution and 5% aqueous ethanol solution. By the above method, a microcarrier for cell culture having an average particle size of 300 um and a specific surface area of 210 cm$^2$/g was produced.

The density, internal porosity, average diameter, and specific surface area of the microcarriers produced in Comparative Examples 1 to 3 are summarized in Table 2 below.

[Table 2]

| Category | Comparative Example 1 | Comparative Example 2 | Comparative Example 3 |
|---|---|---|---|
| Density (g/cm$^3$) | 1.05 | 0.96 | 0.95 |

(continued)

| Category | Comparative Example 1 | Comparative Example 2 | Comparative Example 3 |
|---|---|---|---|
| Internal porosity(%) | 0 | 0 | 9 |
| Average diameter (um) | 250 | 300 | 300 |
| Specific surface area (cm$^2$/g) | 230 | 200 | 210 |
| Remarks | - | No cell adhesion-inducing layer | No primer polymer layer |

**Experimental Example: Measurement of physical properties of microcarrier for cell culture**

For the microcarriers for cell culture produced in Examples and Comparative Examples, the cell culture rate of the microcarriers, the behavior of the microcarriers in the culture medium, and the recovery efficiency of the microcarriers were evaluated by the following methods.

**1. Evaluation of cell culture rate of microcarriers**

The culture medium was filled in a 250 ml spinner flask, and 0.1 g of a microcarrier was added, and the mixture was stirred at 30 rpm. At this time, the temperature of the culture medium was maintained at 37°C, and cultured for 3 days to confirm the cell culture rate of the microcarrier.

**2. Evaluation of the behavior of microcarrier in the culture medium**

The culture medium was filled in a 250 ml spinner flask, and 0.1 g of a microcarrier was added, and the mixture was stirred at 30 rpm. At this time, the temperature of the culture medium was maintained at 37□ and the behavior of the microcarrier was confirmed. The degree of sedimentation of the microcarriers according to the culture time was evaluated.

**3. Evaluation of recovery efficiency of microcarrier**

The cells (chondrocytes) in the culture medium and the microcarriers for cell culture produced in Examples and Comparative Examples were injected and stirred, and the cells were adhered to the microcarriers. After culturing at 37°C for 3 days, the microcarriers to which the cells were adhered were recovered, and then subjected to trypsin treatment to remove the adhered cells, and then, re-dispersed in the culture medium, and the adhered cells were separated in a centrifuge at a rotation speed of 1000 rpm for 5 minutes. Thereafter, the supernatant of the precipitated culture medium was filtered through a 0.2 μm filter to recover microcarriers, which were dried, and then the weight was measured to evaluate the recovery efficiency.

[Table 3]

| | Example 1 | Example 2 | Example 3 | Comparative Example 1 | Comparative Example 2 | Comparative Example 3 |
|---|---|---|---|---|---|---|
| Cell culture rate | - 200% | - 300% | - 400% | ~400% | -150% | -200% |
| Degree of sedimentation | of - | - | - | 100% | - | - |
| Recovery efficiency | 75% | 90% | 95% | 0% | 95% | 95% |

As shown in Table 3, it was confirmed that when the microcarriers produced in Examples 1 to 3 were used, the cells could be easily isolated from the microcarriers after culturing, while maintaining a high cell culture rate compared to Comparative Examples.

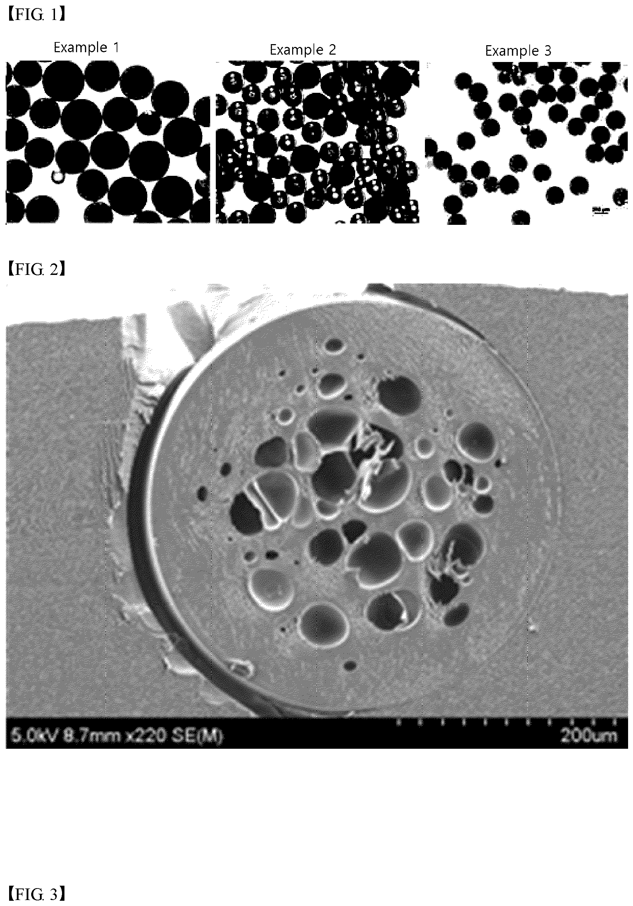

On the other hand, FIG. 2 shows an SEM analysis result of a cross section after foaming of a microcarrier produced in Example 3 of the present disclosure. Through FIG. 2, the cross section of the microcarrier with increased internal porosity after foaming could be confirmed. FIG. 5 shows the results of observing the distribution of the microcarriers produced in Examples 1 to 3 of the present disclosure in the culture medium. Through FIG. 5, it was confirmed that when the density of the microcarrier falls within the range of 0.90-0.98 g/cm$^3$, it is evenly floated in the culture medium, which is advantageous for the cell culture.

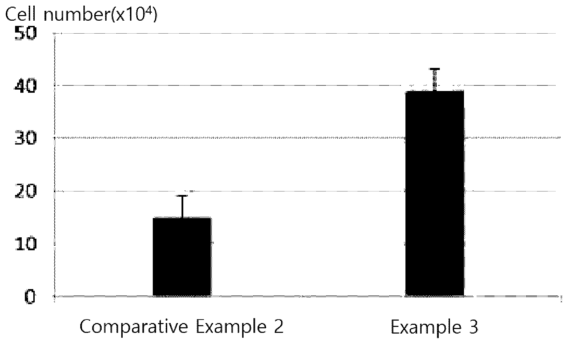

In addition, FIG. 6 shows the results of cell culture of $1 \times 10^4$ chondrocytes for 3 days using 0.2 g of microcarriers produced in Example 3 and Comparative Example 2 of the present disclosure. Through Comparative Example 2, it was confirmed that the cell culture efficiency was significantly reduced when the cell adhesion-inducing layer was not introduced.

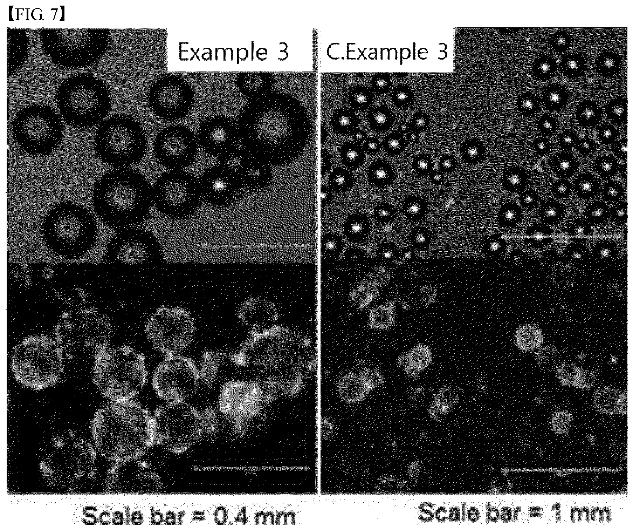

FIG. 7 shows the results of analyzing the cell adhesion shape of the microcarriers produced in Example 3 and Comparative Example 3 of the present disclosure. In the case of Comparative Example 3, it was confirmed that the cell adhesion-inducing layer into which the primer coating layer was not introduced was unstable as compared with Example 3 under the same conditions, so that the cells were not evenly adhered, and the cells could not be efficiently cultured.

**Claims**

1.  A microcarrier for cell culture comprising:

    a foam core containing polystyrene;
    a primer polymer layer formed on a surface of the foam core; and
    a cell adhesion-inducing layer formed on the surface of the primer polymer layer,
    wherein the microcarrier has a density of 0.80 g/cm$^3$ to 0.99 g/cm$^3$.

2.  The microcarrier for cell culture according to claim 1,
    wherein the foam core has a closed cell structure.

3.  The microcarrier for cell culture according to claim 1,
    wherein the foam core has an internal porosity of 2% to 30%.

4.  The microcarrier for cell culture according to claim 1,
    wherein the foam core has a culture medium permeation rate in the range of 0 vol% to 30 vol% of the internal pore volume at the time of culturing cells at 37°C for 72 hours.

5.  The microcarrier for cell culture according to claim 1,
    wherein the primer polymer layer comprises at least one selected from the group consisting of L-dihydroxy phenylalanine, dopamine, norepinephrine, epinephrine, epigallocatechin, and derivatives thereof.

6.  The microcarrier for cell culture according to claim 1,
    wherein the cell adhesion-inducing layer comprises at least one selected from the group consisting of gelatin, collagen, fibronectin, chitosan, polydopamine, poly L-lysine, vitronectin, peptide containing RGD, lignin, cationic dextran, and derivatives thereof.

7.  The microcarrier for cell culture according to claim 1,
    wherein the microcarrier has an average diameter of 50 um to 800 um.

8.  The microcarrier for cell culture according to claim 1,
    wherein the microcarrier has a specific surface area of 50 cm$^2$/g to 2000 cm$^2$/g.

9.  A method for producing a microcarrier for cell culture, the method comprising the steps of:

    heating an aqueous solution containing polystyrene containing a foaming agent;
    foaming the foaming agent to produce a foam core; and
    recovering and drying the produced foam core and then applying a primer polymer layer to a surface of the foam core.

10. The method for producing a microcarrier for cell culture according to claim 9,
    wherein the foam core has a closed cell structure.

**11.** The method for producing a microcarrier for cell culture according to claim 9,
wherein the foam core has an internal porosity of 2% to 30%.

**12.** The method for producing a microcarrier for cell culture according to claim 9,
wherein the primer polymer layer comprises at least one selected from the group consisting of L-dihydroxy phenylalanine, dopamine, norepinephrine, epinephrine, epigallocatechin, and derivatives thereof.

**13.** The method for producing a microcarrier for cell culture according to claim 9,
further comprising a step of coating with a solution containing at least one or more selected from the group consisting of gelatin, collagen, fibronectin, chitosan, polydopamine, poly L-lysine, vitronectin, peptide containing RGD, lignin, cationic dextran, and derivatives thereof after the step of applying a primer polymer layer.

**14.** The method for producing a microcarrier for cell culture according to claim 9,
wherein the foaming agent is selected from the group consisting of a physical foaming agent, a chemical foaming agent, an inorganic foaming agent, and mixtures thereof.

**15.** The method for producing a microcarrier for cell culture according to claim 9,
wherein the step of heating the aqueous solution comprises a step of heat-treating at a temperature of 50°C to 150°C for 1 minute to 10 minutes.

**16.** The method for producing a microcarrier for cell culture according to claim 9,
wherein the step of recovering and drying the produced foam core and then applying the primer polymer layer to the surface of the foam core comprises a step of immersing the foam core in the primer polymer solution for 1 hour to 10 hours.

**17.** The method for producing a microcarrier for cell culture according to claim 13,
wherein the step of coating with the solution comprises:
a step in which a material obtained from the step of recovering and drying the produced foam core and then applying a primer polymer layer to the surface of the foam core is immersed in the solution containing at least one selected from the group consisting of gelatin, collagen, fibronectin, chitosan, polydopamine, poly L-lysine, vitronectin, peptide containing RGD, lignin, cationic dextran, and derivatives thereof for 10 hours to 20 hours.

**18.** A cell culture method using the microcarrier of claim 1.

【FIG. 1】

| Example 1 | Example 2 | Example 3 |

【FIG. 2】

5.0kV 8.7mm x220 SE(M)                              200um

【FIG. 3】

【FIG. 4】

Before foaming

After foaming

After coating

【FIG. 5】

Example 1    Example 2    Example 3

⟶ Particle distribution location

【FIG. 6】

Cell number(x10$^4$)

【FIG. 7】

Example 3 — Scale bar = 0.4 mm

C.Example 3 — Scale bar = 1 mm

## INTERNATIONAL SEARCH REPORT

| | International application No. |
|---|---|
| | **PCT/KR2020/009642** |

**A.    CLASSIFICATION OF SUBJECT MATTER**

**C12N 5/00**(2006.01)i; **C12N 11/08**(2006.01)i

According to International Patent Classification (IPC) or to both national classification and IPC

**B.    FIELDS SEARCHED**

Minimum documentation searched (classification system followed by classification symbols)

C12N 5/00; C08J 7/04; C08J 9/04; C12M 003/04; C12M 1/00; C12N 11/00; C12N 5/074; C12N 11/08

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마이크로 캐리어(micro carrier), 세포 배양(cell culture), 폴리스티렌(polystyrene), 코어(core), 발포(foam)

**C.    DOCUMENTS CONSIDERED TO BE RELEVANT**

| Category* | Citation of document, with indication, where appropriate, of the relevant passages | Relevant to claim No. |
|---|---|---|
| Y | KR 10-2014-0056014 A (INDUSTRY-ACADEMIC COOPERATION FOUNDATION, YONSEI UNIVERSITY) 09 May 2014. See abstract; paragraph [0024]; figure 6; and claims 1-26. | 1-18 |
| Y | US 2002-0155594 A1 (HSIEH, H. V. et al.) 24 October 2002. See abstract; paragraph [0043]; figure 2; and claims 1 and 16-18. | 1-18 |
| A | KR 10-2017-0114993 A (UNIVERSITY-INDUSTRY COOPERATION GROUP OF KYUNG HEE UNIVERSITY) 16 October 2017. See abstract; and claims 1-15. | 1-18 |
| A | US 4994388 A (HILLEGAS, W. J. et al.) 19 February 1991. See abstract; and claims 1-14. | 1-18 |
| A | KR 10-0479218 B1 (PARK, Bong-Kuk) 24 March 2005. See abstract; and claims 1-10. | 1-18 |

☐ Further documents are listed in the continuation of Box C.    ☑ See patent family annex.

| | |
|---|---|
| * Special categories of cited documents: | "T"    later document published after the international filing date or priority date and not in conflict with the application but cited to understand the principle or theory underlying the invention |
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **10 November 2020** | **11 November 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

## INTERNATIONAL SEARCH REPORT
### Information on patent family members

International application No.

**PCT/KR2020/009642**

| Patent document cited in search report | | | Publication date (day/month/year) | Patent family member(s) | | | Publication date (day/month/year) |
|---|---|---|---|---|---|---|---|
| KR | 10-2014-0056014 | A | 09 May 2014 | KR | 10-1545217 | B1 | 18 August 2015 |
| US | 2002-0155594 | A1 | 24 October 2002 | EP | 1245670 | A2 | 02 October 2002 |
| | | | | EP | 1245670 | A3 | 28 January 2004 |
| | | | | JP | 2002-306155 | A | 22 October 2002 |
| KR | 10-2017-0114993 | A | 16 October 2017 | KR | 10-1975100 | B1 | 03 May 2019 |
| | | | | US | 2019-0144820 | A1 | 16 May 2019 |
| | | | | WO | 2017-176055 | A1 | 12 October 2017 |
| US | 4994388 | A | 19 February 1991 | None | | | |
| KR | 10-0479218 | B1 | 24 March 2005 | AU | 2004-314664 | A1 | 11 August 2005 |
| | | | | CA | 2554044 | A1 | 11 August 2005 |
| | | | | CA | 2554044 | C | 07 July 2009 |
| | | | | CN | 100422245 | C | 01 October 2008 |
| | | | | CN | 1906236 | A | 31 January 2007 |
| | | | | EA | 008604 | B1 | 29 June 2007 |
| | | | | EA | 200601401 | A1 | 27 February 2007 |
| | | | | EP | 1709110 | A1 | 11 October 2006 |
| | | | | EP | 1709110 | B1 | 18 March 2020 |
| | | | | JP | 2007-518861 | A | 12 July 2007 |
| | | | | MX | PA06008247 | A | 31 August 2006 |
| | | | | NO | 20063822 | A | 30 October 2006 |
| | | | | UA | 79414 | C2 | 11 June 2007 |
| | | | | US | 2005-0266244 | A1 | 01 December 2005 |
| | | | | US | 7128973 | B2 | 31 October 2006 |
| | | | | WO | 2005-073301 | A1 | 11 August 2005 |

Form PCT/ISA/210 (patent family annex) (July 2019)

**REFERENCES CITED IN THE DESCRIPTION**

**Patent documents cited in the description**

- KR 1020190088576
- KR 1020200090266